# EUROPÄISCHE PATENTSCHRIFT

(11) **EP 2 229 878 B1**
(45) Veröffentlichungstag und Bekanntmachung des Hinweises auf die Patenterteilung: **27.04.2016**
(21) Anmeldenummer: 10165798.9
(22) Anmeldetag: 07.03.2006
(51) Int. Cl.: A61B 5/00

(54) **Akustisches und elektromagnetisches Datenübertragungssystem, integriert in ein Implantat**
Data transmission system using accustical and electromagnetical communication, integrated into an implant
Système de transmission acoustique et électromagnétique, integré dans un implant

(30) Priorität: 31.03.2005 DE 102005014573
(43) Veröffentlichungstag der Anmeldung: 22.09.2010
(62) Teilanmeldung aus: 06004578.8
(73) Patentinhaber: Stryker European Holdings I, LLC, Kalamazoo, MI 49002 (US)
(72) Erfinder: Kaiser, Edgar, 24253, Probsteierhagen (DE); Speitling, Andreas, 24226, Heikendorf (DE)
(74) Vertreter: Schmitz, Alexander

(56) Entgegenhaltungen:
- WO-A-03/043688
- WO-A1-02/094113
- US-A- 4 494 950
- US-A- 6 039 742
- US-A1- 2004 158 299
- US-A1- 2005 026 113

## Beschreibung

Die Erfindung bezieht sich auf ein Meßsystem in Verbindung mit einem Implantat nach dem Patentanspruch 1.

Insbesondere zur Versorgung von Frakturen werden die unterschiedlichsten intramedullären und extramedullären Knochenimplantate, wie Platten, Nägel oder dergleichen eingesetzt. Generell unterscheidet man zwischen Implantaten mit transkutanem Durchgang und solchen, die ohne transkutanen Durchgang implantiert sind. Zu ersteren zählen z.B. externe Fixateure und zu letzteren Nägel oder Platten.

Es ist bekannt, physikalische und/oder chemische Meßgrößen in Verbindung mit einem Implantat zu ermitteln. So ist z.B. erwünscht, die statische und dynamische Belastung von Implantaten zu ermitteln. Aus "Journal of Biomechanics 34" (2001) S. 849-857 ist bekannt, in einem sogenannten Verriegelungs-Knochennagel eine Empfängerspule für den Empfang von externer Energie, eine Meßschaltung, einen Dehnungsmeßstreifen, eine Datenwanderschaltung und eine Sendeschaltung und - spule anzuordnen. Mit Hilfe dieser Elemente sollen die auf den Knochen einwirkenden Kräfte erfaßt werden. Aus "The Journal of Bone and joint Surgery" Volume 83-A Supplement 2, Part 1 (2001) S. 62 -65 ist bekannt, in eine Knieprothese den Dehnungsmeßstreifen einzubauen und diese über Kabel nach außen mit einem Meßgerät zu verbinden. Aus "Medical Engineering & Physics 22" (2000) S. 469 - 479 ist bekannt, z.B. bei einer Oberschenkellasche Dehnungsmeßstreifen anzubringen und diese über Kabel mit einem extrakorporalen Meßgerät zu verbinden. Aus "SPINE" Volume 25, No. 23 pp 2981 - 2986 ist die Kraftmessung an Wirbelsäulenimplantaten bekannt geworden. Aus "Sensors and Actuators" A 97-98 (2002) S. 548-556 sind Belastungsmessungen auch in Verbindung mit Dentalprothesen bekannt geworden.

Bei allen Datenerfassungen in Verbindung mit Implantaten ist die Übermittlung der Meßgrößen nach extern erforderlich. Hierzu werden z.B. Drähte verwendet, welche die Meßeinheit mit einem Meßgerät bzw. einem Meßgrößenverarbeitungsgerät verbinden. Ein solches System ist zwar u.U. für den Implantatträger wenig hinderlich, wenn das Meßgerät außen in angenehmer Weise fixiert wird, die Hindurchführung von Leitungen durch Knochen und Weichgewebe kann jedoch ständige Reizungen verursachen und sogar zu Entzündungen führten. Falls möglich und vom Aufwand her vertretbar, ist die drahtlose Telemetrie, d.h. die drahtlose Übertragung von Meßdaten nach außen als das Mittel der Wahl vorzuziehen. Eine solche drahtlose Übertragung ist etwa aus dem bereits angegebenen Artikel aus "Medical Engineering & Physics" 22 bekannt geworden. Für den Übertragungsweg werden im Nahfeld induktive oder kapazitive Kopplungen mit entsprechenden magnetischen und elektrischen Antennen bei niedrigen Frequenzen verwendet. Werden elektromagnetische Fernfeldantennen verwendet, müssen hohe Frequenzen genutzt werden. Der Übertragungsweg kann sowohl vom Datenerfassungssystem in Richtung des Implantats ("Uplink") als auch vom Implantat in Richtung des Datenerfassungssystems ("Downlink") zeigen. Die Uplink-Strecke wird häufig zur Energieversorgung des implantierten Systems durch induktive Kopplung genutzt.

Ein Nachteil bekannter Telemetriesysteme ist die starke Abschwächung, die elektromagnetische Wellen beim Durchgang durch eine metallische Abschirmung erleiden. Ein weiterer Nachteil besteht in der Schwierigkeit, die Sendeeinheit zu verwirklichen (Miniaturisierung). Besonders nachteilig wirkt sich die Abschirmung aus, wenn zur Übertragung hoher Datenraten hohe Frequenzen über 1 MHz benutzt werden. Da implantierbare Telemetriesysteme aus Gründen der Gewebeverträglichkeit und Wirtschaftlichkeit vorzugsweise in Metallkapseln aus Titan oder Implantatstahl integriert werden, gestaltet sich die Telemetrie problematisch.

US 4,494,950 offenbart ein System mit einer Mehrzahl von separaten Modulen, welche gemeinsam einen nützlichen biomedizinischen Zweck erfüllen. Alle Module können intrakorporal oder extrakorporal am Körper angeordnet sein. Signale werden von einem Modul zu einem anderen mittels elektromagnetischer Wellen, elektrischer Signale, die den Körper als elektrischen Leiter nutzen, oder mittels akustischer Wellen gesendet.

WO 03/043688 A1 offenbart ein System und ein Verfahren zum Kommunizieren mit einem Implantat innerhalb eines Körpers unter Verwendung von akustischer Telemetrie, mit einer externen Kommunikationsvorrichtung, die an der Haut des Körpers anbringbar ist.

Der Erfindung liegt die Aufgabe zugrunde, ein Meßsystem in Verbindung mit einem Implantat zu schaffen, das eine Übertragung von Daten problemlos ermöglicht.

Diese Aufgabe wird durch die Merkmale des Patentanspruchs 1 gelöst.

Es ist zwar denkbar, für eine Telemetrie in Verbindung mit einem Implantat die Energiequelle mit zu implantieren und auf eine externe Energieversorgung und eine externe Steuerung zu verzichten. Dies dürfte normalerweise nur eine Ausnahme sein. Typisch ist die Verwendung eines Uplinks und eines Downlinks. Der Uplink mit einer externen Sendeeinheit und einer internen intrakorporalen Empfangseinheit dient üblicherweise dazu, Steuersignale auf die Meßeinheit und auch auf den Sender für den Downlink zu übertragen. Ferner wird auf diese Weise die Energieversorgung sichergestellt, insbesondere durch induktive Kopplung. Die intrakorporale Sendeeinheit für Downlink ist z. B. vollständig metallgekapselt und kommuniziert mit einer extrakorporalen externen Empfangseinheit. Mithin liegen zwei drahtlose Übertragungsstrecken vor. Erfindungsgemäß ist zumindest die Übertragungsstrecke zwischen der internen Sendeeinheit und der externen Empfangseinheit für eine akustische Datenübermittlung ausgelegt. Die von der Meßeinheit ermittelten Daten werden im Downlink-Empfänger in akustische Daten umgewandelt bzw. zur Ansteuerung eines akustischen Wandlers verwendet zur Übertragung auf die extrakorporale Empfangseinheit. Es ist zwar denkbar, auch für die andere Übertragungsstrecke (Uplink) auf eine akustische Übertragung zurückzugreifen; dies könnte für die Steuerdaten sinnvoll sein, während für die elektrische Energieversorgung von außen die elektromagnetische Einkopplung vorzuziehen ist.

Der Vorteil des erfindungsgemäßen Meßsystems liegt darin, daß akustische Schwingungen auch metallische Abschirmungen mit geringer Dämpfung durchdringen können. Akustische Wellen können sich mit niedriger Dämpfung auch in metallischen Implantaten ausbreiten. Daher können das Implantat selbst, aber auch Knochen- und Weichteilgewebe des Implantatträgers als Übertragungsmedium für die akustische Telemetrie genutzt werden. Nach einer Ausgestaltung der Erfindung ist es daher vorteilhaft, wenn die interne Sendeeinheit akustisch an das Implantat angekoppelt ist. Bei einer intrakorporalen Anordnung des Implantats kann die externe Sendeeinheit und/oder die akustische Empfangseinheit zur Anbringung auf der Haut ausgebildet sein. So kann z.B. ein diese Teile aufnehmendes Gehäuse in geeigneter Weise an dem betreffenden Körperteil befestigt werden. Eine akustische Sendeeinheit kann auch in nicht metallischer Kapselung leicht miniaturisiert werden.

Bei einem Implanat mit transkutanem Durchgang, wie z.B. bei einem externen Fixateur, können die metallischen Stifte des Fixateurs die Übertragungsstrecke bilden und der akustische Empfänger im distalen Bereich der Stifte oder an den Anbringungspunkten angebracht werden.

Schließlich ist nach einer Ausgestaltung der Erfindung auch möglich, die Meßeinheit und die interne Sende- und Empfangseinheit an einem intramedulären Nagel anzubringen und die externe akustische Empfangseinheit lösbar am zugekehrten Ende des Nagels bzw. an einem mit dem Nagel verbindbaren Eintreib- und/oder Zielinstrument anzubringen.

Die Erfindung bezieht sich auch auf einen sogenannten Transponder, der implantiert wird und dessen Energieversorgung mittels induktiver und/oder kapazitiver Kopplung entlang des Uplinks erfolgt. Der Downlink, d. h. die Übertragungsstrecke vom Transponder zu einer extrakorporalen Empfangseinheit kann auf akustischem Wege erfolgen. Es ist bekannt, die Datenübertragung zu einem Transponder (Uplink) und umgekehrt (Downlink) mit der sogenannten RFID-Technik zu realisieren. Diese hat jedoch den Nachteil, daß sie leicht gestört werden kann bzw. ihrerseits Störungen verursacht, z. B. in Sicherheitssystemen. Es können z. B. Fehlalarme ausgelöst werden. Auch die Zuverlässigkeit der Datenauslesung wird durch einen akustischen Downlink erhöht, da eine elektromagnetische Übertragungsstrecke leicht gestört werden kann. Ferner können höhere Anforderungen bezüglich Datensicherheit und Persönlichkeitsschutz erfüllt werden, da der Downlink nur hergestellt werden kann, wenn der externe akustische Empfänger in direktem Kontakt mit der Haut gebracht wird. Ein Auslesen von Daten aus der Entfernung ist nicht möglich.

Bei einer Ausgestaltung der Erfindung kann der implantierbare Transponder eine selbständige Einheit bilden oder in ein Implantat integriert sein. Er kann nach einer weiteren Ausgestaltung der Erfindung in einer implantierbaren Telemetrieeinheit integriert oder mit einer solchen gekoppelt sein. Die Datenübertragung von einer externen Sendeeinheit zum implantierten Transponder kann nach einer weiteren Ausgestaltung der Erfindung durch elektromagnetischen Schwingungen und/oder Wellen erfolgen. Der Uplink kann jedoch ebenfalls als akustische Strecke ausgebildet sein.

Ein Transponder kann entweder nur zur Wiedergabe fest eingespeicherter Daten (read-only transponder) vorgesehen werden, z. B. um eine Artikel- oder Seriennummer eines Implantats abzufragen oder auch einen modifizierbaren Speicher enthalten, der ganz oder teilweise durch eine externe Einheit beschrieben werden kann, z. B. zur Speicherung und Wiedergabe von Patientendaten.

Die Erfindung soll nachfolgend anhand von zwei in Zeichnungen dargestellten Ausführungsbeispielen näher erläutert werden.
- Fig. 1: zeigt eine schematische Darstellung mit einem Telemetriesystem nach der Erfindung,
- Fig. 2: zeigt eine andere schematische Darstellung einer Anwendung eines Telemetriesystems nach der Erfindung.

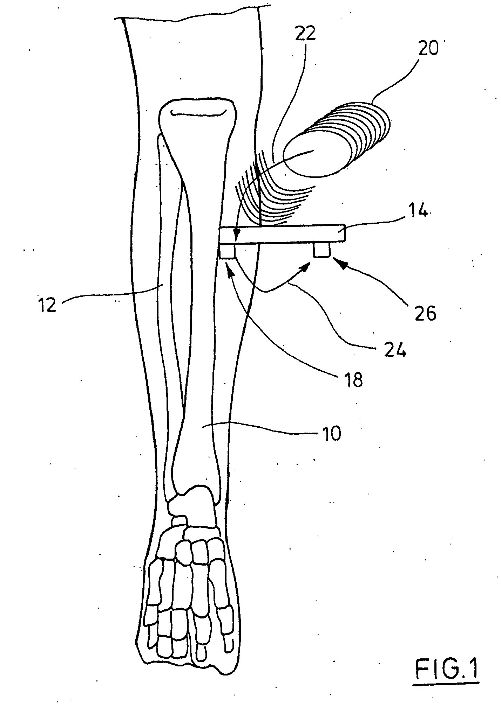

In Fig. 1 ist schematisch ein menschlicher Unterschenkel dargestellt mit Schienbein 10 und Wadenbein 12. Es ist eine Fraktur des Schienbeins 10 unterstellt, welche mit einem Fixateur extern versorgt ist. Dieser ist im einzelnen nicht dargestellt. Ersatzweise ist lediglich bei 14 ein Stift dargestellt, wie er üblicherweise bei derartigen Geräten verwendet wird. Bekanntlich wird in jedes Fraktursegment mindestens ein derartiger Stift eingeschraubt, welcher Stifte außerhalb des Beins über ein geeignetes Gestänge gegeneinander verspannt werden, um die Knochenfragmente gegeneinander zu positionieren. Da eine derartige Versorgung notorisch ist, soll hierauf im einzelnen nicht eingegangen werden.

Am Stift 14 ist nahe des Schienenbeins 10 eine Einheit 18 angebracht. Sie enthält eine elektromagnetische Empfangseinheit und eine akustische Sendeeinheit. Beide sind in einem metallischen Gehäuse gekapselt. Die akustische Sendeeinheit ist über einen geeigneten Wandler an den Stift 14 angekoppelt. Die Energie für die Einheit 18 wird elektrisch von einem elektromagnetischen Sender 20 eingekoppelt. Mithin liegt ein elektromagnetischer Uplink 22 vor.

Mit der Empfangs- und Sendeeinheit 18 ist eine nicht gezeigte Meßeinheit gekoppelt, die entweder in dem Gehäuse der Einheit 18 untergebracht oder mit dieser verbunden ist und welche gewünschte Daten physikalischer und/oder chemischer Natur erfaßt, beispielsweise Daten, welche Auskunft geben über den Heilungsverlauf der Fraktur, über die dynamische Belastung des Knochens usw. Die gemessenen Daten werden in geeigneter Weise aufbereitet, so daß sie mit Hilfe der akustischen Sendeeinheit und des akustischen Wandlers auf den Stift 14 gekoppelt werden können. Der Stift bildet mithin die akustische Übertragungsstrecke (Downlink 24). Am distalen Ende des Stiftes 14 sitzt ein akustischer Empfänger 26, der die vom akustischen Sender kommenden Daten empfängt. Sie können dann in geeigneter Weise durch einen Datenprozessor oder dergleichen aufbereitet werden.

In der Ausführungsform nach Fig. 1 ist der akustische Wandler des externen Downlinkempfängers 26 an einer Komponente außerhalb des Körpers angebracht, einem transkutanem Pin 14. Die akustische Telemetrieübertragung erfolgt ausschließlich durch das Material des Implantatsystems. Alternativ ist möglich, den akustischen Wandler des externen Downlink-Empfängers an der Körperoberfläche (Haut) anzubringen. Die akustische Telemetriebübertragung erfolgt dann durch das Implantatmaterial, Knochen- und Weichteilgewebe und durch die Haut.

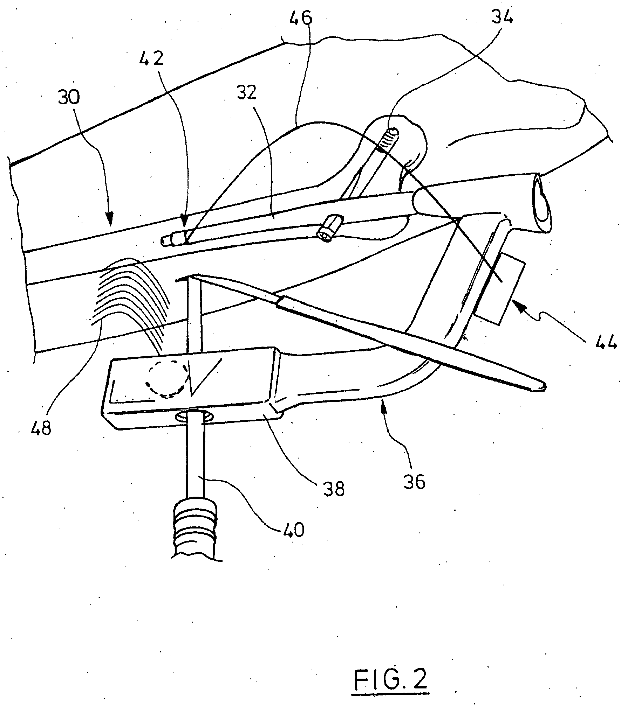

In Fig. 2 ist der Implantiervorgang eines sogenannten Gammanagels in den proximalen Femur 30 gezeigt. Der Nagel besteht aus einem Verriegelungsnagel 32 und einem schräg durch diesen gesteckten Oberschenkelhalsstift 34. Der Verriegelungsnagel 32 wird mit Hilfe eines Zielgeräts 36, das am proximalen Nagelende fest angebracht ist, eingetrieben. Es dient mithin auch als Einschlagelement. Am Zielarm 38 des Zielinstruments 36 sind Bohrungen vorgesehen zum Auffinden der Querlöcher im Nagel 32. Eine entsprechende Bohrhülse ist bei 40 dargestellt. Das beschriebene Implantiersystem ist allgemein bekannt und soll nicht weiter beschrieben werden.

Im distalen Endbereich des Nagels 32 ist eine Empfangs- und Sendeeinheit 42 angebracht, vergleichbar der Empfangs- und Sendeeinheit 18 nach Fig. 1. Sie enthält eine oder ist verbunden mit einer Meßeinheit am oder im Nagel zur Messung von Daten, die für den operierenden Arzt von Interesse sind. Am Zielinstrument 36 ist bei 44 eine akustische Empfangseinheit angebracht. Während der Operation kommuniziert der akustische Sender in der Einheit 42 mit dem akustischen Empfänger 44 über den Nagel 32 und den Zielbügel des Instruments 36. Diese Teile werden mithin als akustische Wellenleiter verwendet. Dies ist durch 46 (Downlink) angedeutet. Die Energieversorgung erfolgt über einen elektromagnetischen Uplink 48. Auch die Steuerungsdaten für die Einheit 32 bzw. die Meßeinheit können elektromagnetisch übertragen werden.

Im Allgemeinen ist ein Datenübertragungssystem in Verbindung mit einem Implantat, gemäß einer Ausführungsform gekennzeichnet durch einen implantierbaren Transponder mit einer internen Sendeeinheit, einer extrakorporal angeordneten Empfangseinheit, einer ersten Übertragungsstrecke (Downlink) zwischen interner Sende-und extrakorporaler Empfangseinheit, und einer elektromagnetischen Energieversorgung mittels induktiver und/oder kapazitiver Kopplung entlang einer Uplink-Strecke, wobei die erste Übertragungsstrecke mit akustischen Schwingungen bzw. Wellen arbeitet.

Gemäß einer anderen Ausführungsform ist ein Datenübertragungssystem in Verbindung mit einem Implantat, gekennzeichnet durch eine mit dem Implantat implantierbare Messeinheit für die Messung mindestens einer physikalischen oder chemischen Messgrösse am Implantat oder im Bereich von diesem, eine mit der Messeinheit integrierte oder koppelbare und implantierbare interne Sendeeinheit, eine extrakorporal angeordnete Empfangseinheit, eine erste Übertragungsstrecke (Downlink) zwischen der internen Sende- und der extrakorporalen Empfangseinheit, eine extrakorporal angeordnete externe Sendeeinheit, eine von der externen Sendeeinheit ansteuerbare implantierbare Empfangseinheit, und eine zweite Übertragungsstrecke (Uplink) zwischen der externen Sende- und internen Empfangseinheit, über die die Messeinheit und die interne Sendeeinheit angesteuert wird, wobei die erste Übertragungsstrecke mit akustischen Schwingungen bzw. Wellen arbeitet.

Die zweite Übertragungsstrecke dieses Datenübertragungssystems kann mit elektromagnetischen Wellen arbeiten, wobei über die zweite Übertragungsstrecke sowohl Daten als auch Energie für den Betrieb der Messeinheit und der internen Empfangs- und Sendeeinheit übertragen werden können. Hierbei kann eine dritte Übertragungsstrecke zur Übertragung von Daten und akustischen Schwingungen bzw. Wellen arbeiten.

Gemäß einem Aspekt können die Meßeinheit und die interne Empfangs- und Sendeeinheit des Datenübertragungssystems in einem körperverträglichen Material gekapselt sein.

Gemäß einem weiteren Aspekt kann die interne Sendeeinheit des Datenübertragungssystems akustisch an das Implantat angekoppelt sein.

Gemäß einem weiteren Aspekt können die externe Sendeeinheit und/oder die akustische externe Empfangseinheit des Datenübertragungssystems zur Ankopplung auf der Haut ausgebildet sind.

Gemäß einem weiteren Aspekt des Datenübertragungssystems kann bei einem Implantat mit transkutanem Durchgang mittels eines metallischen Stifts oder dergleichen dieser die erste Übertragungsstrecke bilden.

Gemäß einem weiteren Aspekt können die Messeinheit und die interne Sende- und Empfangseinheit des Datenübertragungssystems an einem intramedullären Nagel angebracht sein und die externe akustische Empfangseinheit lösbar am zugeordneten Ende des Nagels bzw. an einem mit dem Nagel verbundenen Eintreibinstrument anbringbar sein.

## Patentansprüche

1. Datenübertragungssystem mit:
- einem Knochenimplantat (32),
- einem implantierbaren Transponder mit einer internen Sendeeinheit (42),
- einer extrakorporal angeordnete Empfangseinheit (44),
- einer erste Übertragungsstrecke (46) zwischen der internen Sendeeinheit und der extrakorporalen Empfangseinheit,
- einer elektromagnetische Energieversorgung mittels induktiver und/oder kapazitiver Kopplung entlang einer Uplink-Strecke (48),
wobei die erste Übertragungsstrecke mit akustischen Schwingungen bzw. Wellen arbeitet,
charakterisiert dadurch, dass:
- der implantierbare Transponder in das Knochenimplantat integriert ist, und
- die erste Übertragungsstrecke mit akustischen Schwingungen bzw. Wellen entlang des Knochenimplantats arbeitet.

2. Datenübertragungssystem nach Anspruch 1, **dadurch gekennzeichnet, dass** der implantierbare Transponder in einer implantierbaren Telemetrieeinheit integriert oder mit einer solchen gekoppelt ist.

3. Datenübertragungssystem nach einem der Ansprüche 1 bis 2, **dadurch gekennzeichnet, dass** eine Datenübermittlung von der externen Sendeeinheit zum implantierten Transponder mittels elektromagnetischer Schwingungen und/oder Wellen erfolgt.

4. Datenübertragungssystem nach einem der Ansprüche 1 bis 3, **dadurch gekennzeichnet, dass** die Datenübermittlung von externer Sendeeinheit zum implantierten Transponder mittels akustischer Schwingungen und/oder Wellen erfolgt.
KK:KMS:nah

5. Datenübertragungssystem nach einem der Ansprüche 1 bis 4, **dadurch gekennzeichnet, dass** die implantierte akustische Sendeeinheit an Knochen, Implantat oder Weichteilgewebe akustisch gekoppelt ist.

6. Datenübertragungssystem nach einem der Ansprüche 1 bis 5, **dadurch gekennzeichnet, dass** die externe akustische Sende- und/oder Empfangseinheit an die Haut oder einen externen Teil eines transkutenen Implantats gekoppelt ist.

7. Datenübertragungssystem nach einem der Ansprüche 1 bis 6, **dadurch gekennzeichnet, dass** der implantierte Transponder einen Speicher mit fest eingespeicherten Daten enthält (read-only transponder).

8. Datenübertragungssystem nach einem der Ansprüche 1 bis 7, **dadurch gekennzeichnet, dass** der implantierte Transponder einen modifizierbaren beschreibbaren Speicher enthält.

## Claims

1. Data transmission system comprising:
- a bone implant (32),
- an implantable transponder comprising an internal transmission unit (42),
- a receiving unit (44), arranged extracorporeally,
- a first transmission path (46) between the internal transmission unit and the extracorporeal receiving unit,
- an electromagnetic power supply by means of inductive and/or capacitive coupling along an uplink path (48),
the first transmission path operating by means of acoustic vibrations or waves, **characterised in that**
- the implantable transponder is integrated in the bone implant, and
- the first transmission path operates by means of acoustic vibrations or waves along the bone implant.

2. Data transmission system according to claim 1, **characterised in that** the implantable transponder is integrated in an implantable telemetry unit or is coupled to a telemetry unit of this type.

3. Data transmission system according to any of claims 1 to 2, **characterised in that** data is transmitted from the external transmission unit to the implanted transponder by means of electromagnetic vibrations and/or waves.

4. Data transmission system according to any of claims 1 to 3, **characterised in that** data is transmitted from the external transmission unit to the implanted transponder by means of acoustic vibrations and/or waves.

5. Data transmission system according to any of claims 1 to 4, **characterised in that** the implanted acoustic transmission unit is acoustically coupled to bone, an implant or soft tissue.

6. Data transmission system according to any of claims 1 to 5, **characterised in that** the external acoustic transmission unit and/or receiving unit is coupled to the skin or to an external part of a transcutaneous implant.

7. Data transmission system according to any of claims 1 to 6, **characterised in that** the implanted transponder contains a memory having data which is stored in a read-only manner (read-only transponder).

8. Data transmission system according to any of claims 1 to 7, **characterised in that** the implanted transponder contains a modifiable, writable memory.

## Revendications

1. Système de transmission de données, avec
- un implant osseux (32),
- un transpondeur implantable avec une unité d'émission interne (42),
- une unité de réception (44) disposée de façon extracorporelle,
- un premier trajet de transmission (46) entre l'unité d'émission interne et l'unité de réception extracorporelle,
- une alimentation en énergie électromagnétique au moyen d'un couplage inductif et/ou capacitif le long d'un trajet de liaison montante (48),
dans lequel le premier trajet de transmission travaille avec des vibrations ou respectivement ondes acoustiques,
**caractérisé en ce que** :
- le transpondeur implantable est intégré dans l'implant osseux, et
- le premier trajet de transmission travaille avec des vibrations ou respectivement ondes acoustiques le long de l'implant osseux.

2. Système de transmission de données selon la revendication 1, **caractérisé en ce que** le transpondeur implantable est intégré dans une unité de télémesure implantable ou est couplé à une telle unité.

3. Système de transmission de données selon l'une des revendications 1 à 2 , **caractérisé en ce qu'**une communication de données à partir de l'unité d'émission externe vers le transpondeur implanté s'effectue au moyen de vibrations et/ou ondes électromagnétiques.

4. Système de transmission de données selon l'une des revendications 1 à 3, **caractérisé en ce que** la communication de données à partir de l'unité d'émission externe vers le transpondeur implanté s'effectue au moyen de vibrations et/ou ondes acoustiques.

5. Système de transmission de données selon l'une des revendications 1 à 4, **caractérisé en ce que** l'unité d'émission acoustique implantée est couplée acoustiquement à un os, un implant ou un tissu mou.

6. Système de transmission de données selon l'une des revendications 1 à 5, **caractérisé en ce que** l'unité d'émission et/ou de réception acoustique externe est couplée à la peau ou à une partie externe d'un implant transcutané.

7. Système de transmission de données selon l'une des revendications 1 à 6, **caractérisé en ce que** le transpondeur implanté contient une mémoire avec des données enregistrées de façon fixe (read-only transponder).

8. Système de transmission de données selon l'une des revendications 1 à 7, **caractérisé en ce que** le transpondeur implanté contient une mémoire inscriptible modifiable.
